# EUROPÄISCHE PATENTSCHRIFT

(11) **EP 1 000 603 B1**
(45) Veröffentlichungstag und Bekanntmachung des Hinweises auf die Patenterteilung: **09.02.2005**
(21) Anmeldenummer: 99117912.8
(22) Anmeldetag: 13.09.1999
(51) Int. Cl.: A61K 7/00, A61K 7/48

(54) **Verwendung von oberflächenaktiven Citronensäureestern zur Stabilisierung von Flavonen, Flavanonen bzw. Flavonoiden, synergistische Gemische aus Flavonen, Flavanonen bzw. Flavonoiden und oberflächenaktiven Substanzen sowie kosmetische und dermatologische Zubereitungen mit einem Gehalt an solchen Gemischen**
Use of surface-active citric acid esters for stabilizing flavones, flavanones or flavonoids, and synergistic mixtures, cosmetic and dermatological preparations containing them
Utilisation d'esters tensioactifs d'acide citrique pour stabiliser de flavones, flavanones ou de flavonoides et mélanges synergiques, compositions cosmétiques et dermatologiques les contenant

(30) Priorität: 01.10.1998 DE 19845305
(43) Veröffentlichungstag der Anmeldung: 17.05.2000
(73) Patentinhaber: Beiersdorf Aktiengesellschaft, 20245 Hamburg (DE)
(72) Erfinder: Max, Heiner, Dr., 22297 Hamburg (DE); Schönrock, Uwe, Dr., 23886 Nahe (DE); Stäb, Franz, Dr., 21379 Echem (DE); Untiedt, Sven, Dr., 20259 Hamburg (DE)

(56) Entgegenhaltungen:
- EP-A- 0 980 684
- DE-A- 4 242 876
- DE-A- 4 444 238
- DE-A- 19 802 204
- DE-A- 19 802 205
- DE-A- 19 807 774
- FR-A- 2 723 316

## Beschreibung

Die vorliegende Erfindung betrifft die Verwendung an sich bekannter oberflächenaktiver Substanzen zur Stabilisierung von α-Glucosylrutin gegenüber chemischen Abbaureaktionen, insbesondere photochemischen Abbaureaktionen und/oder oxidationsbedingten Abbaureaktionen.

Darüberhinaus betrifft die Erfindung synergistische Gemische aus α-Glucosylrutin und Glycerylstearatcitrat sowie kosmetische und dermatologische Zubereitungen mit einem Gehalt an solchen Gemischen. Bevorzugt betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft dementsprechend in bevorzugten Ausführungsformen kosmetische bzw. dermatologische dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Die menschliche Haut übt als größtes Organ des Menschen zahlreiche lebenswichtige Funktionen aus. Mit durchschittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist.

Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Die Verwendung von Flavonen bzw. Flavonoiden in der Kosmetik bzw. Dermatologie ist an sich bekannt. So beschreibt die DE-OS 44 44 238 Kombinationen von Zimtsäurederivaten und Flavonglycosiden, beispielsweise α-Glycosylrutin als Antioxidantien und als Wirkstoffe gegen andere Indikationen.

Das Patentdokument EP-A-0980684 offenbart eine Zusammensetzung enthaltend 2.5% Glycerylstearatcitrat und 0.5% alpha-Glucosylrutin.

Es war indes überraschend und für den Fachmann nicht vorherzusehen, daß Wirkstoffkombinationen aus einem oder mehrerem partiell neutralisierten Estern von Monoglyceriden und/oder Diglyceriden gesättigter Fettsäuren mit Citronensäure und α-Glucosylrutin den Nachteilen des Standes der Technik abhilft.

Erfindungsgemäß ist ferner die Verwendung von einem oder mehrerem partiell neutralisierten Estern von Monoglyceriden und/oder Diglyceriden gesättigter Fettsäuren mit Citronensäure zur Stabilisierung von Flavonen, Flavanonen bzw. Flavonoiden gegenüber chemischen Abbaureaktionen, insbesondere photochemischen Abbaureaktionen und/oder oxidationsbedingten Abbaureaktionen.

Flavon und seine Derivate (oft auch kollektiv Flavone" genannt) sind durch folgende Grundstruktur gekennzeichnet (Substitutionspostitionen angegeben):

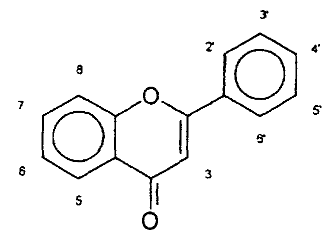

Einige der wichtigeren Flavone, welche auch in der belebten Natur aufzufinden sind, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Flavonoide sind Glycoside der Flavone, der Flavanone, deren Grundgerüst durch die folgende Struktur gekennzeichnet ist: 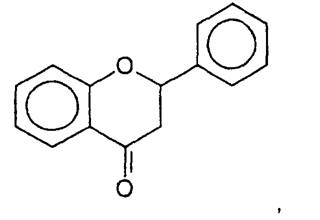 der 3-Hydroxyflavone (Flavonole), deren Grundgerüst durch die folgende Struktur gekennzeichnet ist: 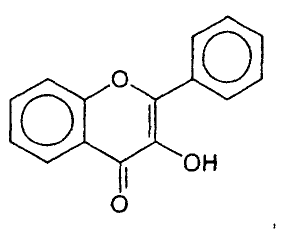 der Aurone, deren Grundgerüst durch die folgende Struktur gekennzeichnet ist: 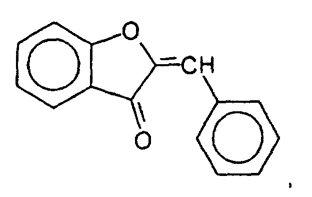 sowie der Isoflavone, deren Grundgerüst durch die folgende Struktur gekennzeichnet ist:

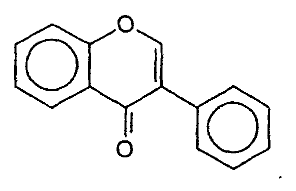

Das erfindungsgemäße Flavonoid ist α-Glucosylrutin. Es zeichnet sich durch folgende Struktur aus:

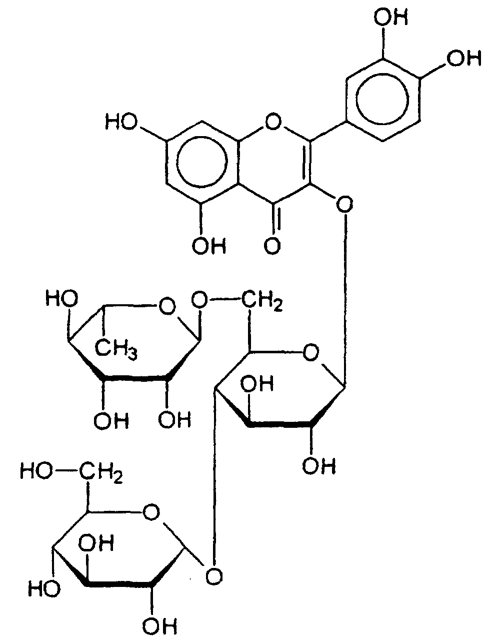

Das α-Glucosylrutin ist erfindungsgemäß vorteilhaft in kosmetischen oder dermatologischen Zubereitungen bevorzugt zu 0.01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Ein besonders vorteilhafter partiell neutralisierter Ester von Monoglyceriden und/oder Diglyceriden gesättigter Fettsäuren mit Citronensäure ist das Glycerylstearatcitrat. Solche Citronensäureester sind beispielsweise erhältlich unter der Produktbezeichnung IMWITOR® 370" der Gesellschaft Hüls AG.

Die Gesamtmenge an den erfindungsgemäß verwendeten partiell neutralisierten Estern von Monoglyceriden und/oder Diglyceriden gesättigter Fettsäuren mit Citronensäure in den fertigen kosmetischen oder dermatologischen Zubereitungen wird vorteilhaft aus dem Bereich von 0,1 - 20 Gew.-%, bevorzugt 0,5 - 10,0 Gew.-% insbesondere 2,0 - 5,0 Gew.-% gewählt, bezogen auf das Gesamtgewicht der Zubereitungen.

Es ist von Vorteil, Gewichtsverhältnisse zwischen mindestens einem Flavonderivat und/oder Flavanonderivat, insbesondere mindestens einem Flavonoid einerseits und mindestens einem partiell neutralisierten Ester von Monoglyceriden und/oder Diglyceriden gesättigter Fettsäuren mit Citronensäure andererseits aus dem Bereich von 1 : 1 bis 1 : 200, bevorzugt 1 : 5 bis 1 zu 100, insbesondere 1 : 50 zu wählen.

Die erfindungsgemäße Kombination aus α Glucosylrutin und mindestens einem partiell neutralisierten Ester von Monoglyceriden und/oder Diglyceriden gesättigter Fettsäuren mit Citronensäure wird im Rahmen dieser Schrift auch kollektiv als "erfindungsgemäßer Wirkstoff" oder "erfindungsgemäß verwendeteter Wirkstoff" oder erfindungsgemäß verwendetete Wirkstoffkombination" bezeichnet bzw. mit sinnverwandten Bezeichnungen belegt.

Die Schrift JP-OS Hei-06-138,941 beschreibt zwar orale Zubereitungen mit einem Gehalt an wasserlöslichen Glycosiden, welche beispielsweise gewählt werden können aus der Gruppe α-Glucosylrutin, α-Glucosylmyricetin, α-Glucosylisoquercitrin und α-Glucosylquercitrin. Die Schrift JP-OS Hei-04-363,395 beschreibt ein Verfahren, die Zersetzung von Parfümbestandteilen zu verhindern, welche sich unter anderem durch einen Zusatz an α-Glucosylrutin zu den entsprechenden Zubereitungen auszeichnet. Ferner beschreiben die Schriften EP-OS 586 303 und EP-OS 595 694 die Verwendung von Flavonoiden als Antioxidantien bzw. Lichtschutzsubstanzen in Kosmetika.

Kein Hinweis ist diesen Schriften aber zu entnehmen, welcher in die Richtung der vorliegenden Erfindung weisen könnte.

Es war für den Fachmann daher nicht vorauszusehen gewesen, daß die erfindungsgemäß verwendeten Wirkstoffkombinationen bzw. kosmetische oder dermatologische Zubereitungen, solche enthaltend
- besser als Antioxidans wirken
- besser als Radikalfänger wirken
- besser die Bindung von schädlichen Photoprodukten an Lipide, DNS und Proteine verhindern
- besser gegen die Hautalterung wirken
- besser die Haut gegen Photoreaktionen schützen
- besser entzündlichen Reaktionen vorbeugen würde
als die Wirkstoffe, Wirkstoffkombinationen und Zubereitungen des Standes der Technik. Ferner war nicht vorauszusehen gewesen, daß die erfindungsgemäß verwendeten Wirkstoffkombinationen in kosmetischen oder dermatologischen Zubereitungen höhere Stabilität aufweist als die jeweils einzeln verwendeten Wirkstoffe, was insbesondere das α-Glucosylrutin betrifft.

Erfindungsgemäß sind daher die Verwendung von Wirkstoffkombinationen aus α-Glucosylrutin und mindestens einem partiell neutralisierten Ester von Monoglyceriden und/oder Diglyceriden gesättigter Fettsäuren mit Citronensäure als Antioxidans sowie seine Verwendung zur Bekämpfung und/oder Prophylaxe der durch oxidative Beanspruchung hervorgerufenen Hautalterung und entzündlicher Reaktionen.

Als besonders vorteilhafte Ausführungsform der vorliegenden Erfindung wird ferner angesehen die Verwendung von Wirkstoffkombinationen aus α-Glucosylrutin und mindestens einem partiell neutralisierten Ester von Monoglyceriden und/oder Diglyceriden gesättigter Fettsäuren mit Citronensäure zur Bekämpfung und/oder Prophylaxe des oxidativen Stresses.

Die erfindungsgemäßen kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,1 Gew.-% bis 20 Gew.-%, bevorzugt 0,5 Gew.-% bis 10 Gew.-%, insbesondere 2,0 - 5,0 Gew.-%, bezogen auf das Gesamtgewicht der Zubereitungen, an erfindungsgemäß verwendeten Wirkstoffkombinationen.

Es ist erfindungsgemäß bevorzugt, den erfindungsgemäß verwendeten Wirkstoffkombinationen bzw. kosmetischen oder dermatologischen Zubereitungen, solche Wirkstoffkombinationen enthaltend Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Emulsion oder Mikroemulsion vom Typ Öl-in-Wasser (O/W), eine multiple Emulsionen, beispielsweise vom Typ Wasser-in-Öl-in-Wasser (W/O/W), ein Gel oder einen festen Stift darstellen. Es ist auch vorteilhaft, die Flavonoide in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, erfindungsgemäß verwendete Wirkstoffkombinationen in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können erfindungsgemäß verwendete Wirkstoffkombinationen auch mit anderen Antioxidantien und/oder Radikalfängern kombiniert werden.

Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, lsopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Atkytbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrigalkoholischen oder alkoholischen Gelen vorzugweise ein Polyacrylat ist.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z. B.:
- 3-Benzylidencampher-Derivate, vorzugsweise 3-(4-Methylbenzyliden)campher, 3-Benzylidencampher;
- 4-Aminobenzoäsäure-Derivate, vorzugsweise 4-(Dimethylamino)-benzoesäure(2-ethylhexyl)ester, 4-(Dimethylamino)benzoäsäureamylestec
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Wirkstoffkombinationen mit mindestens einem UVB-Filter als Antioxidans bzw. die Verwendung einer Kombination der erfindungsgemäß verwendeten Wirkstoffkombinationen mit mindestens einem UVB-Filter als Antioxidans in einer kosmetischen oder dermatologischen Zubereitung.

Es kann auch von Vorteil sein, die erfindungsgemäß verwendeten Wirkstoffkombinationen mit UVA-Filtem zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination von erfindungsgemäß verwendeten Wirkstoffkombinationen mit mindestens einem UVA-Filter als Antioxidans bzw. die Verwendung einer Kombination der erfindungsgemäßen Wirkstoffkombinationen mit mindestens einem UVA-Filter als Antioxidans in einer kosmetischen oder dermatologischen Zubereitung.

Gegenstand der Erfindung ist auch die Verwendung einer Kombination aus erfindungsgemäß verwendeten Wirkstoffkombinationen mit mindestens einem UVA-Filter und mindestens einem UVB-Filter als Antioxidans bzw. die Verwendung einer Kombination aus erfindungsgemäßen Wirkstoffen mit mindestens einem UVA-Filter und mindestens einem UVB-Filter als Antioxidans in einer kosmetischen oder dermatologischen Zubereitung.

Kosmetische und dermatologische Zubereitungen mit einem wirksamen Gehalt an erfindungsgemäß verwendeten Wirkstoffkombinationen können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine anionische, nichtionische oder amphotere oberflächenaktive Substanz, oder auch Gemische aus solchen Substanzen, die erfindungsgemäß verwendeten Wirkstoffkombinationen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäß verwendete Wirkstoffkombinationen in wirksamer Konzentration. Die Menge der verwendeten Polymeren liegt z.B. zwischen 0,1 und 10 Gew.-%, bevorzugt zwischen 0,1 und 3 Gew.-%.

Erfindungsgemäß können kosmetische Zubereitungen zur Behandlung und Pflege der Haare als Gele vorliegen, die neben einem wirksamen Gehalt an erfindungsgemäßen Wirkstoffen und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge an erfindungsgemäßem Wirkstoff in einem für die Haare bestimmten Mittel 0,1 Gew.-% bis 10 Gew.-%, insbesondere 0,5 Gew.-% bis 5 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobemsteinsäurehalbester
- Alkylethercarboxylate
- Eiweiß-Fettsäure-Kondensate
- Alkylbetaine und Amidobetaine
- Fettsäurealkanolamide
- Polyglycoiether-Derivate

Kosmetische Zubereitungen, die kosmetische Reinigungszubereitungen für die Haut darstellen, können in flüssiger oder fester Form vorliegen. Sie enthalten neben erfindungsgemäß verwendeten Wirkstoffkombinationen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an erfindungsgemäßem Wirkstoff vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Pertglanzagentien, Pflanzenextrakte, Vitamine, Wirkstoffe und dergleichen.

Die vorliegende Erfindung umfaßt auch ein kosmetisches Verfahren zum Schutze der Haut und der Haare vor oxidativen bzw. photooxidativen Prozessen, das dadurch gekennzeichnet ist, daß man ein kosmetisches Mittel, welches eine wirksame Konzentration an erfindungsgemäß verwendeten Wirkstoffkombinationen enthält, in ausreichender Menge auf die Haut oder Haare aufbringt.

Vorzugsweise beträgt die Menge an erfindungsgemäß verwendeten Wirkstoffkombinationen in diesen Zubereitungen 0,1 - 20 Gew.-%, bevorzugt 0,5 - 10 Gew.-%, insbesondere 2,0 - 5,0 Gew.-%, bezogen auf das Gesamtgewicht der Zubereitungen.

Gegenstand der Erfindung ist auch das Verfahren zur Herstellung der erfindungsgemäßen kosmetischen Mittel, das dadurch gekennzeichnet ist, daß man in an sich bekannter Weise erfindungsgemäßen Wirkstoffkombinationen in kosmetische und dermatologische Formulierungen einarbeitet.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1 (O/W-Creme):

| | Gew.-% |
|---|---|
| Glycerylstearatcitrat | 3,60 |
| Sorbitanmonostearat | 1,20 |
| Cetylstearylalkohol | 1,20 |
| Octyldodecanol | 3,00 |
| Caprylsäure-/Caprinsäuretriglyceride | 3,00 |
| Dicaprylylether | 3,00 |
| α-Glucosylrutin | 0,20 |
| Na₃HEDTA | 0,20 |
| Xanthangummi | 0,10 |
| Glycerin | 3,00 |
| Parfüm, Konservierungsmittel, Farbstoffe, Antioxidantien | q.s. |
| Wasser | ad 100,00 |

### Beispiel 2 (O/W-Creme):

| | Gew.-% |
|---|---|
| Glycerylstearatcitrat | 2,40 |
| Sorbitanmonostearat | 0,80 |
| Cetylstearylalkohol | 0,80 |
| Octyldodecanol | 3,00 |
| Caprylsäure-/Caprinsäuretriglyceride | 3,00 |
| Dicaprylylether | 3,00 |
| Xanthangummi | 0,10 |
| Citronensäure | 0,10 |
| Natriumcitrat | 0,20 |
| α-Glucosylrutin | 0,30 |
| Glycerin | 3,00 |
| Parfüm, Konservierungsmittel, Farbstoffe, Antioxidantien | q.s. |
| Wasser | ad 100,00 |

### Beispiel 3 (Emulgatorgel):

| | Gew.-% |
|---|---|
| Glycerylstearatcitrat | 3,60 |
| Sorbitanmonostearat | 1,20 |
| Cetylstearylalkohol | 1,20 |
| Xanthangummi | 0,10 |
| α-Glucosylrutin | 0,20 |
| Glycerin | 3,00 |
| Parfüm, Konservierungsmittel, Farbstoffe, Antioxidantien | q.s. |
| Wasser | ad 100,00 |

## Patentansprüche

1. Verwendung von einem oder mehrerem partiell neutralisierten Estern von Monoglyceriden und/oder Diglyceriden gesättigter Fettsäuren mit Citronensäure zur Stabilisierung von α-Glucosylrutin gegenüber chemischen Abbaureaktionen, insbesondere photochemischen Abbaureaktionen und/oder oxidationsbedingten Abbaureaktionen.

2. Verwendung nach Anspruch 1, **dadurch gekennzeichnet, daß** das α-Glucosylrutin in einem wirksamen Gehalt in kosmetischen oder dermatologischen Zubereitungen vorliegt.

3. Verwendung nach Anspruch 2, **dadurch gekennzeichnet, daß** daß das α-Glucosylrutin in Konzentrationen von 0,01 - 10 Gew.-%, bevorzugt 0,05 - 5 Gew.-%, insbesondere 0,1 - 2,0 Gew.-% bezogen auf das Gesamtgewicht der Zubereitungen, vorliegt.

4. Verwendung nach Anspruch 1, **dadurch gekennzeichnet, daß** als partiell neutralisierter Ester von Monoglyceriden und/oder Diglyceriden gesättigter Fettsäuren mit Citronensäure das Glycerylstearatcitrat gewählt wird.

5. Verwendung nach Anspruch 2, **dadurch gekennzeichnet, daß** der oder die partiell neutralisierten Ester von Monoglyceriden und/oder Diglyceriden gesättigter Fettsäuren mit Citronensäure in Konzentrationen von 0,1 - 20 Gew.-%, bevorzugt 0,5 - 10 Gew.-%insbesondere 2,0 - 5,0 Gew.-% bezogen auf das Gesamtgewicht der Zubereitungen, vorliegen.

6. Kosmetische Zubereitungen enthaltend α-Glucosytrutin und Glycerylstearatcitrat, mit Ausnahme einer Zubereitung, die
| | Gew.-% |
|---|---|
| Paraffinöl | 4.00 |
| Schibutter | 4,00 |
| Rizinusöl | 1,00 |
| Squalan | 4,00 |
| Caprylsäure/Caprinsäuretriglyceride | 4.00 |
| Glycerylstearatcitrat | 2,50 |
| Cetylalkohol | 4.00 |
| Xanthan Gummi | 0,50 |
| α-Glucosylrutin | 0.50 |
| Octylmethoxycinnamat | 2,00 |
| TiO₂ | 1,00 |
| Natriumlactat | 3,00 |
| Glycerin | 3,00 |
| Konservierungsmittel und Parfüm | q.s. |
| Wasser | ad 100,00 |
enthält.

7. Zubereitungen nach Anspruch 6, **dadurch gekennzeichnet, daß** daß das α-Glucosylrutin in kosmetischen oder topischen dermatologischen Zubereitungen in Konzentrationen von 0,01 - 10 Gew.-%, bevorzugt 0,05 - 5 Gew.-%, insbesondere 0,1 - 2,0 Gew.-% bezogen auf das Gesamtgewicht der Zubereitungen, vorliegt.

8. Zubereitungen nach Anspruch 6, **dadurch gekennzeichnet, daß** das Glycerylstearatcitrat in kosmetischen oder topischen dermatologischen Zubereitungen in Konzentrationen von 0,1 - 20 Gew.-%. bevorzugt 0,5 -10 Gew.-%, insbesondere 2,0 - 5,0 Gew.-% bezogen auf das Gesamtgewicht der Zubereitungen, vorliegt.

## Claims

1. Use of one or more partially neutralized esters of monoglycerides and/or diglycerides of saturated fatty acids with citric acid for stabilizing α-glucosylrutin against chemical degradation reactions, in particular photochemical degradation reactions and/or oxidation-induced degradation reactions.

2. Use according to Claim 1, **characterized in that** the α-glucosylrutin is present in an effective content in cosmetic or dermatological preparations.

3. Use according to Claim 2, **characterized in that** the α-glucosylrutin is present in concentrations of 0.01-10% by weight, preferably 0.05-5% by weight, in particular 0.1-2.0% by weight, based on the total weight of the preparations.

4. Use according to Claim 1, **characterized in that** the glyceryl stearate citrate is chosen as partially neutralized esters of monoglycerides and/or diglycerides of saturated fatty acids with citric acid.

5. Use according to Claim 2, **characterized in that** the partially neutralized ester(s) of monoglycerides and/or diglycerides of saturated fatty acids with citric acid is/are present in concentrations of 0.1-20% by weight, preferably 0.5-10% by weight, in particular 2.0-5.0% by weight, based on the total weight of the preparations.

6. Cosmetic preparations comprising α-glucosylrutin and glyceryl stearate citrate, with the exception of a preparation which comprises
| | % by wt. |
|---|---|
| Paraffin oil | 4.00 |
| Shea butter | 4.00 |
| Castor oil | 1.00 |
| Squalane | 4.00 |
| Caprylic/capric triglycerides | 4.00 |
| Glyceryl stearate citrate | 2.50 |
| Cetyl alcohol | 4.00 |
| Xanthan gum | 0.50 |
| α-Glucosylrutin | 0.50 |
| Octyl methoxycinnamate | 2.00 |
| TiO₂ | 1.00 |
| Sodium lactate | 3.00 |
| Glycerol | 3.00 |
| Preservative and perfume | q.s. |
| Water | ad 100.00 |

7. Preparations according to Claim 6, **characterized in that** the α-glucosylrutin is present in cosmetic or topical dermatological preparations in concentrations of from 0.01-10% by weight, preferably 0.05-5% by weight, in particular 0.1-2.0% by weight, based on the total weight of the preparations.

8. Preparations according to Claim 6, **characterized in that** the glyceryl stearate citrate is present in cosmetic or topical dermatological preparations in concentrations of 0.1-20% by weight, preferably 0.5-10% by weight, in particular 2.0-5.0% by weight, based on the total weight of the preparations.

## Revendications

1. Utilisation d'un ou de plusieurs esters, partiellement neutralisés à l'aide d'acide citrique, de monoglycérides et/ou de diglycérides d'acides gras saturés, en vue de la stabilisation du α-glucosylrutinoside vis-à-vis des réactions de dégradation chimiques, en particulier de réactions de dégradation photochimiques et/ou de réactions de dégradation contrôlées par oxydation.

2. Utilisation selon la revendication 1, **caractérisée en ce que** l'α-glucosylrutinoside est présent dans une teneur efficace dans des préparations cosmétiques ou dermatologiques.

3. Utilisation selon la revendication 2, **caractérisée en ce que** l'alpha-glucosylrutinoside est présent dans des concentrations de 0,01 - 10% en poids, de préférence, de 0,05 - 5% en poids, en particulier de 0,1 - 2,0% en poids, par rapport au poids total des préparations.

4. Utilisation selon la revendication 1, **caractérisée en ce que** l'on sélectionne le stéarate-citrate de glycéryle en tant qu'ester, partiellement neutralisé à l'aide d'acide citrique, de monoglycérides et/ou de diglycérides d'acides gras saturés.

5. Utilisation selon la revendication 2, **caractérisée en ce que** le ou les esters, partiellement neutralisés à l'aide d'acide citrique, de monoglycérides et/ou de diglycérides d'acides gras saturés est ou sont présents dans des concentrations de 0,1 - 20% en poids, de préférence, de 0,5 - 10% en poids, en particulier de 2,0 - 5,0% en poids, par rapport au poids total des préparations.

6. Préparations cosmétiques, contenant de l'alpha-glycosylrutinoside et du citrate-stéarate de glycéryle, à l'exception d'une préparation, qui contient
| | % en poids |
|---|---|
| Huile de paraffine | 4,00 |
| Beurre de karité | 4,00 |
| Huile de ricin | 1,00 |
| Squalane | 4,00 |
| Triglycéride de l'acide caprylique/ de l'acide caprique | 4,00 |
| Stéarate-citrate de glycéryle | 2,50 |
| Alcool cétylique | 4,00 |
| Gomme de xanthane | 0,50 |
| Alpha-glucosylrutinoside | 0,50 |
| Méthoxycinnamate d'octyle | 2,00 |
| Ti0₂ | 1,00 |
| Lactate de sodium | 3,00 |
| Glycérine | 3,00 |
| Agents de conservation et parfums | Au choix |
| | |
| Eau (complément à 100%) | 100,00 |

7. Préparations selon la revendication 6, **caractérisé en ce que** l'alpha-glucosylrutinoside est présent dans les préparations cosmétiques ou dermatologiques dans des concentrations de 0,01 - 10% en poids, de préférence, de 0,05 - 5% en poids, en particulier de 0,1 - 2,0% en poids, par rapport au poids total des préparations.

8. Préparations selon la revendication 6, **caractérisé en ce que** le stéarate-citrate de glycéryle est présent dans les préparations cosmétiques ou topiques dermatologiques dans des concentrations de 0,1 - 20% en poids, de préférence, de 0,5 - 10% en poids, en particulier de 2,0 - 5,0% en poids, par rapport au poids total des préparations.
